# EUROPEAN PATENT SPECIFICATION

(11) **EP 0 604 931 B1**
(45) Date of publication and mention of the grant of the patent: **02.03.2005**
(21) Application number: 93120881.3
(22) Date of filing: 24.12.1993
(51) Int. Cl.: A61N 5/067

(54) **Medical laser apparatus and diagnosing/curing apparatus using the medical laser apparatus**
Medizinischer Laser und dessen Verwendung in einem Gerät für Diagnose und Therapie
Laser médical et appareil pour le diagnostic et la thérapie utilisant ce laser

(30) Priority: 28.12.1992 JP 34778492; 24.08.1993 JP 20932593
(43) Date of publication of application: 06.07.1994
(73) Proprietor: MATSUSHITA ELECTRIC INDUSTRIAL CO., LTD., Kadoma-shi, Osaka 571-8501 (JP)
(72) Inventor: Aizawa, Katsuo, Yokohama-shi, Kanagawa-ken (JP); Ii, Yoshiteru, Osaka-shi, Osaka-fu (JP); Kaneda, Akira, Toyono-gun, Osaka-fu (JP); Yuzu, Takayoshi, Ikoma-gun, Nara-ken (JP); Yamamoto, Toshiyoshi, Sanda-shi, Hyogo-ken (JP); Kato, Harubumi, Tokyo-to (JP)
(74) Representative: Eisenführ, Speiser & Partner

(56) References cited:
- EP-A- 0 429 297
- WO-A-84/04665
- GB-A- 2 007 015
- US-A- 4 932 934
- US-A- 4 994 059

## Description

### BACKGROUND OF THE INVENTION

The present invention relates to a medical laser apparatus to be used as a light source for a diagnosing/curing apparatus which treats a focus of a tumor such as a cancer or the like through irradiation of light to the focus. When the light having the wavelength agreed with the absorption wavelength of a photosensitizer which has affinity to the focus and has been preliminarily accumulated in the focus is irradiated to the focus, the photosensitizer is excited, making it possible to diagnose or treat the focus. The present invention relates alike to a diagnosing/curing apparatus using the medical laser apparatus.

In accordance with the development of the electronic medical-care technology, the photodynamic diagnosis (referred to as PDD hereinbelow) and the photodynamic therapy (referred to as PDT hereinafter) both utilizing the laser light have made a rapid progress recently. Specifically speaking of PDD and PDT, while a photosensitizer having affinity to a tumor and displaying a photochemical reaction, e.g., an emission of fluorescence or a cellcidal action has been accumulated in a focus of the tumor beforehand, the light is irradiated to the focus, which induces the excitation of the photosensitizer, thereby to diagnose the focus by measuring the emitted fluorescence (PDD) or treat the focus by the cellcidal action (PDT). It is better that the wavelength of the light irradiated to the focus is agreed with the absorption wavelength of the photosensitizer in order to efficiently excite the photosensitizer, and therefore a laser light source has been generally employed as a light source of the light irradiating to the focus. In this case, the laser light source is fitted to the absorption wavelength of the using photosensitizer.

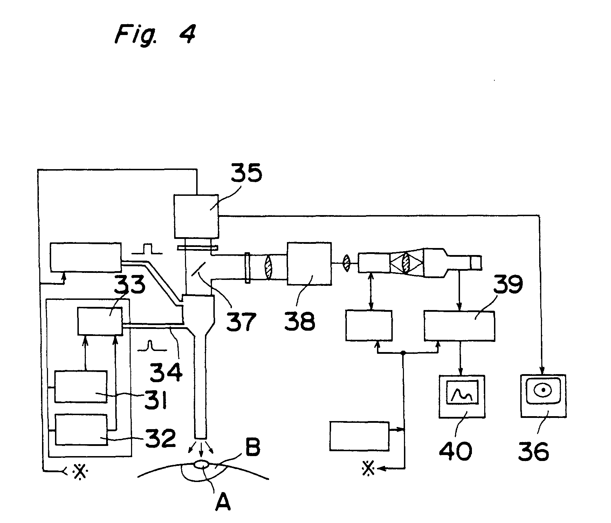

A dye laser which uses hematoporphyrin as a photosensitizer and an excimer laser as a laser light source (referred to as an excimer dye laser hereinbelow) has been often used in the above-described type of diagnosing/curing apparatus for treating cancers, as is discussed in Japanese Patent Publication Nos. 63-2633 (2633/1988) and 63-9464 (9464/1988). Now, the conventional diagnosing/curing apparatus using the laser device disclosed in the publication Nos. 63-2633 and 63-9464 will be described with reference to Fig. 4.

Fig. 4 schematically shows the constitution of a diagnosing/curing apparatus of cancers using a conventional laser apparatus. In Fig. 4, A is a focus of a cancer and B indicates the peripheral part of the focus A where hematoporphyrin has been absorbed as a photosensitizer beforehand. A first pulse source 31 for diagnosing purpose and a second pulse source 32 for treating purpose are both constituted of an excimer dye laser. An excimer dye laser for exciting the first and second dye lasers 31, 32 repeatedly oscillates with the oscillating wavelength 308nm and the pulse width 30ns while varying the energy in the range of several mJ-100mJ. The oscillating wavelength of the first pulse source 31 is 405nm and that of the second pulse source 32 is 630nm. The first and second pulse sources 31, 32 are switched by a switching part 33. The other references represent respectively: 34 a light transmission line; 35 a TV camera; 36 a TV monitor; 37 a half mirror; 38 a spectroscope; 39 a spectrum analyzing part; and 40 a display unit.

The diagnosing/curing apparatus in the above-described constitution operates in the manner as follows.

When a cancer is to be diagnosed, a laser light of the wavelength 405nm generated from the first pulse light source 31 is irradiated to the focus A and the peripheral part B through the switching part 33 and the light transmission line 34. An image of fluorescence of the wavelength 630nm, 690nm excited by the laser light of 405nm wavelength is photographed by the TV camera 35 and displayed for observation on the screen of the TV monitor 36. At the same time, the image of fluorescence is taken out by the half mirror 37 and divided by the spectroscope 38. The spectrum is analyzed in the spectrum analyzing part 39 and the wavelength of the spectrum is displayed by the display unit 40. In order to treat the cancer, then, a laser light of the wavelength 630nm produced by the second pulse light source 32 is, through the switching part 33 and the light transmission line 34, irradiated to the focus A. The mode is subsequently switched to the diagnosing mode again thereby to confirm the result of the treatment. The cancer is diagnosed and cured by repeatedly switching the modes as above.

Not only because the fluorescence peculiar to hematoporphyrin is efficiently excited by the light of the wavelength 405nm, but because the adverse influences by the scattering light can be restricted owing to the far difference of the wavelengths 630nm and 690nm of fluorescence, the first pulse light source 31 for diagnosing purpose uses the wavelength 405nm. Meanwhile, the reason why the second pulse light source 32 for curing purpose is set at the wavelength 630nm is that the laser light of the wavelength 630nm is transmitted well through the tissue and efficiently absorbed in hematoporphyrin.

In addition to the aforementioned example, the photosensitizers in (Table 1) below are proposed for use in PDD and PDT and also the lasers shown in (Table 1) are tried to be used as a laser light source for PDT.

**(Table 1)**

| Photosensitizer | Absorption wavelength [nm] | Laser light source (projection wavelength [nm]) | Disadvantages of laser devices |
|---|---|---|---|
| HpD | 630 | Excimer dye laser Argon dye laser (624±6.5nm) | *Deterioration of solution of coloring matter is fast *Bulky and expensive |
| | | Gold vapor laser (627.8nm) | *Necessary to warm up for 30 min. or more *Life of gas and oscillating tube is short *Bulky and expensive. |
| PH-1126 | 650 | Krypton laser (647nm) | *Life of gas is short *Bulky and expensive |
| NPe6 | 664 | Argon dye laser (667±5nm) | *Deterioration of solution of coloring matter is fast *Bulky and expensive |

It is a drawback of the conventional diagnosing/curing apparatus of cancers that the wavelength of the projected laser light is hard to control.

In other words, it is necessary to make the wavelength of the laser light agreed with the absorption band of the photosensitizer so as to efficiently excite the photosensitizer. Generally, the gas laser (Table 1) is impossible to meet the absorption band of a plurality of the photosensitizers. What's worse, it is difficult for the gas laser to have the wavelength agreed with the maximum absorption wavelength of even a single photosensitizer. Although a dye laser as depicted with reference to the above conventional example has been employed to solve the problem, it is necessary to exchange the solution of a coloring matter in order to change the oscillating wavelength of the dye laser. Therefore, a plurality of dye lasers corresponding to a plurality of different kinds of solutions of a coloring matter should be prepared and exchanged for every wavelength if the wavelength of the laser light is required to be changed, for instance, when the using photosensitizer is changed or when the wavelength of the laser light is changed at the curing time from that at the diagnosing time.

In the case where the dye laser is used, therefore, the diagnosing/curing apparatus becomes disadvantageously bulky in size to accommodate a plurality of kinds of solutions of a coloring matter and a switching part of the solutions.

A second disadvantage of the diagnosing/curing apparatus using the dye laser is that the solution of a coloring matter of the dye laser is easy to deteriorate, inviting the change of the wavelength of the resultant laser light or the decrease of the output. Since the safety of the laser light is an essential and indispensable condition to ensure the effect of PDD and especially PDT, it is a great problem of the dye laser that the solution of the coloring matter should be exchanged or a circulator of the coloring matter should be cleaned frequently. Further, the wavelength of the laser light is undesirably changed or the laser output is decreased during the irradiation if the solution used in the dye laser is easy to degrade, that is, the irradiating condition of the laser light should be set with such changes in the wavelength or output as above taken into consideration and, the change of the laser light should be arranged to be detected.

Thirdly, when the wavelength is converted by the dye laser, the full width at half maximum (FWHM) of the wavelength of the obtained laser light expands to at least 10nm or so. If the full width at half maximum is wide, the laser light increasingly shifts from the absorption band of the photosensitizer, thus worsening the exciting efficiency of the photosensitizer. Although it may be arranged to reduce the full width at half maximum of the dye laser by using a band pass filter or a diffraction grating, only the excessive component is cut, but the exciting efficiency is left unimproved.

A fourth drawback is the poor converting efficiency of energy of the dye laser when the wavelength is converted. Therefore, the excimer laser, etc. used as a light source to excite the dye laser is required to generate a high output in order to achieve enough energy from the converted laser light. In other words, the conventional medical laser apparatus and the diagnosing/curing apparatus of cancers using the conventional medical laser apparatus are liable to be bulky and expensive.

A fifth drawback inherent in the prior art necessitates two light sources for diagnosing purpose and for curing purpose as well as the switching part to switch the light sources. The apparatus consequently is bulky and expensive and moreover, inconvenient to switch the light sources, with an erroneous manipulation feared.

### SUMMARY OF THE INVENTION

The object of the present invention is therefore to provide, with eliminating the aforementioned drawbacks of the conventional apparatuses, a compact and inexpensive medical laser apparatus which achieves laser light of the oscillating wavelength fit for a plurality of kinds of photosensitizers and also a plurality of exciting conditions thereof, and is maintenance-easy with a narrow full width at half maximum and good exciting efficiency.

A further object of the present invention is to provide a diagnosing/curing apparatus using the medical laser apparatus which realizes both diagnosis and treatment by a single light source thereby to make a diagnosis simultaneously during the treatment.

In accomplishing these and other objects, according to the present invention, there is provided a medical laser apparatus as defined in claim 1. Preferred embodiments are defined in the dependent claims.

### BRIEF DESCRIPTION OF THE DRAWINGS

These and other objects and features of the present invention will become clear from the following description taken in conjunction with the preferred embodiments thereof with reference to the accompanying drawings, in which:
Fig. 1 is a block diagram showing the constitution of a medical laser apparatus according to a first embodiment of the present invention;
Fig. 2 is a block diagram showing the constitution of a diagnosing/curing apparatus according to a second embodiment of the present invention;
Fig. 3 is a characteristic diagram of a band pass filter used in the second embodiment of the present invention; and
Fig. 4 is a block diagram showing the constitution of a diagnosing/curing apparatus of cancers using a conventional laser apparatus.

### DETAILED DESCRIPTION OF THE PREFERRED EMBODIMENTS

Before the description of the present invention proceeds, it is to be noted that like parts are designated by like reference numerals throughout the accompanying drawings.

### [First Embodiment]

A medical laser apparatus according to a first embodiment of the present invention will be discussed hereinbelow with reference to the accompanying drawings.

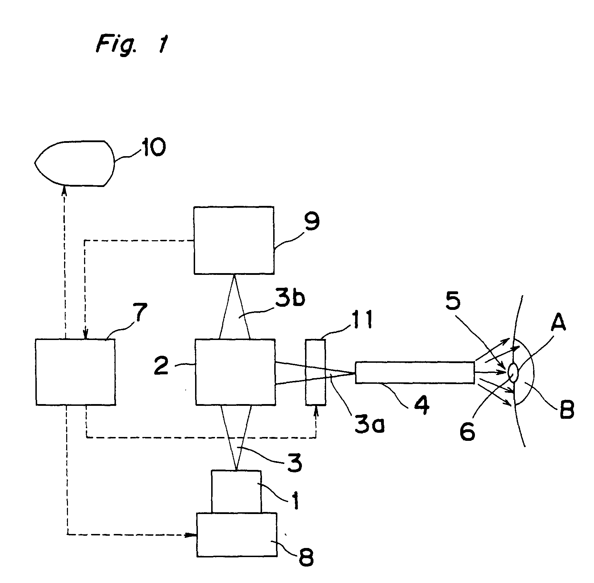

The constitution of a medical laser apparatus according to a first embodiment of the present invention is shown in Fig. 1. Referring to Fig. 1, an AlGaInP semiconductor laser 1 assures of the oscillating frequency 664nm, full width at half maximum (FWHM) of ±1nm, temperature characteristic of the oscillating wavelength 0.2nm/deg, and operable temperature range -100 through +80°C during driving at 0°C. An optical system 2 separates a laser light 3 projected from the semiconductor laser 1 to an irradiating laser light 3a and a wavelength detecting laser light 3b. A photosensitizer 6 is preliminarily administered in a target portion to be treated 5 including a focus A and a peripheral part B of the focus A. The other reference numerals indicate: 4 an optical fiber; 7 a control unit; 8 a temperature controlling device; 9 a wavelength detecting unit for detecting the wavelength of the laser light 3b; 10 a wavelength displaying unit; and 11 a shutter working as an automatic irradiation stopping unit in association with the control unit 7.

The operation of the medical laser apparatus in the constitution as above will be described below.

The wavelength of the laser light 3 projected from the semiconductor laser 1 is determined by the temperature of the semiconductor laser 1. That is, when the temperature of the semiconductor laser 1 is varied in the range of -100 through +80°C by the temperature controlling device 8, the wavelength of the laser light 3 is changed within 644 through 680nm. Accordingly, the wavelength of the laser light 3 is obtained which is suited to the absorption wavelength of the using photosensitizer 6 and the purpose of the treatment.

In the instant embodiment, NPe6 (trade name of Nippon Petrochemical Co., Ltd.) of a chlorin group in (Table 1) is used as the photosensitizer 6. The temperature of the semiconductor laser 1 is set at 0°C when the laser light 3 of the center wavelength 664nm in the absorption band of 660nm through 668nm of the photosensitizer 6 is desired. On the other hand, the temperature of the semiconductor laser 1 is controlled to be -15°C in order to obtain the laser light of the shorter wavelength 660nm in the absorption band for an aim to be described later. When the laser light 3 of the center wavelength 650nm and the shorter wavelength 644nm in the absorption band of 647nm through 653nm is to be obtained with the use of PH-1126 (trade name of Hamari Chemicals, Ltd.) of a pheophorbide group (Table 1), the semiconductor laser is controlled to be -70°C and -100°C, respectively. The full width at half maximum is narrower than a width of the absorption band, where an energy absorption of the photosensitizer is equal to or more than 90% of the maximal value in the vicinity of the oscillating wavelength.

Since the full width at half maximum is ±1nm, the wavelength of the laser light 3 projected from the semiconductor laser 1 controlled at the temperature 0°C, - 15°C, -70°C, and -100°C is 663-665nm, 659-661nm, 649-651nm, and 643-645nm, that is, the energy of the laser light 3 is held within the absorption band of the using photosensitizer 6.

A part of the laser light 3 from the controlled semiconductor laser 1 is separated by the optical system 2 and guided to the wavelength detecting unit 9 as the wavelength detecting laser light 3b. The detecting unit 9 detects the wavelength of the laser light 3b. The detected result is sent to the control unit 7. It is decided by the control unit 7 whether the laser light 3b matches a predetermined condition to control the wavelength thereof. The detecting result and the value of the wavelength is displayed by the wavelength displaying unit 10. The control unit 7 makes the automatic irradiation stopping unit 11 start to work in case the laser light 3b does not match the predetermined controlling condition so as to shut the irradiating laser light 3a.

When the laser light 3 conforms to the predetermined controlling condition, the shutter 11 is opened and the irradiating laser light 3a is condensed into the optical fiber 4 to be irradiated to the target portion 5 from an end of the optical fiber 4.

As described hereinabove, the oscillating wavelength of the laser is controlled thereby to obtain the laser light of the wavelength of the narrow full width at half maximum which is suited to the absorption wavelength of a plurality of kinds of photosensitizers and the purpose of the treatment, so that the photosensitizers can be excited efficiently. Moreover, the laser apparatus is almost maintenance-free, compact in size and inexpensive.

### [Second Embodiment]

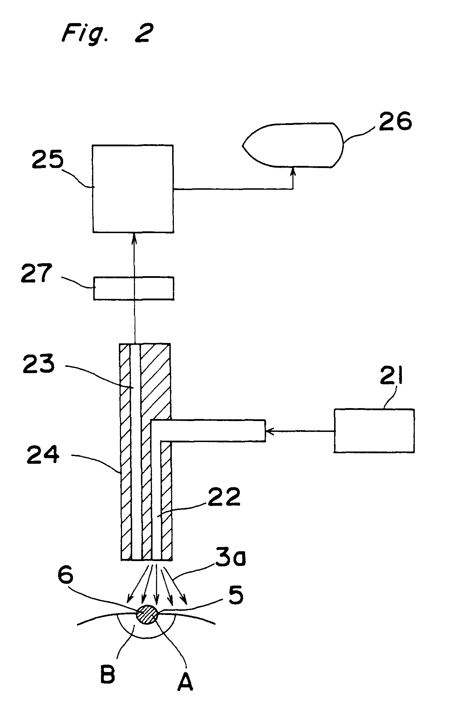

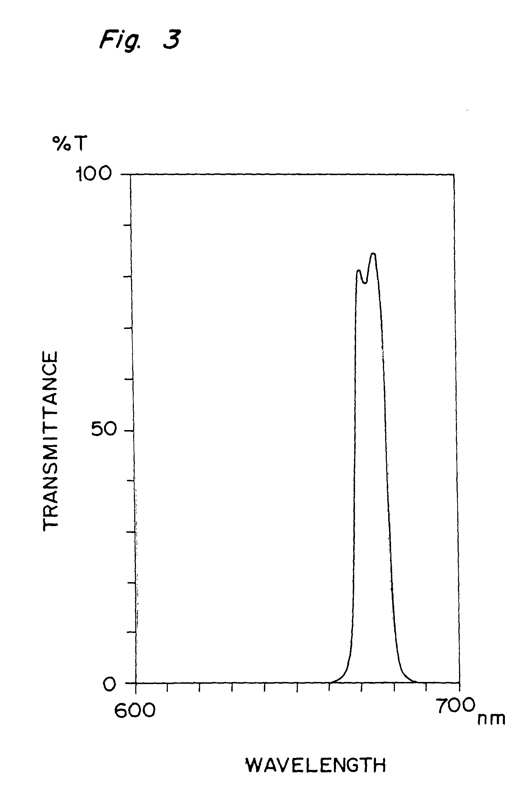

A diagnosing/curing apparatus of cancers according to a second embodiment of the present invention will be discussed with reference to Figs. 2 and 3.

Fig. 2 is a block diagram showing the constitution of the diagnosing/curing apparatus of cancers according to the second embodiment of the present invention. In Fig. 2, reference numeral 21 denotes a laser light source which is the medical laser apparatus using the semiconductor laser disclosed in the foregoing first embodiment, 22 denotes a light transmission line through which the irradiating laser light 3a from the laser light source 21 is introduced to the vicinity of the focus, and 23 denotes an image transmission line through which an image of fluorescence is transmitted to observe the focus and the periphery of the focus. A light guiding device 24 incorporating the light transmission line 22 and the image transmission line 23 guides both the lines 22 and 23 to the vicinity of the focus. An image-picking-up/analyzing unit 25 picks up images in the vicinity of the focus through the image transmission line 23 and analyzes the images. The analyzing result is displayed by an image displaying unit 26. A band-pass filter 27 with a considerably narrow band width. i.e., approximately ±3nm to the designated wavelength is composed of a dielectric multi-layer film (for example, a total dielectric interference filter DIF of Vacuum Optics Corporation of Japan having the characteristic shown in Fig. 3). The band-pass filter 27 allows only the light of the wavelength in the vicinity of that of fluorescence of the using photosensitizer separately from the irradiating laser light 3a to pass. The passing wavelength is approximately 670nm when the photosensitizer of a chlorin group is used, or approximately 654nm when the photosensitizer of a pheophorbide group is used. The band-pass filter 27 is placed on an optical axis connecting the image transmission line 23 with the image-picking-up/analyzing unit 25. It is to be noted here that the band-pass filter 27 can be equipped with a plurality of band-pass filters respectively corresponding to a plurality of photosensitizers, and also a switching means (not shown) to switch the band-pass filters. The remaining reference numerals, for instance, 5 and the like represent the same parts as in Fig. 1.

The operation of the diagnosing/curing apparatus of cancers constituted in the above-described arrangement will be depicted below.

In the first place, the irradiating laser light 3a projected from the laser light source 21 is irradiated via the light transmission line 22 to the target portion 5 where the photosensitizer has been preliminarily accumulated. At this time, the laser light 3a is controlled by the temperature controlling device to attain the center wavelength of the absorption band of the photosensitizer so that the treatment results in the optimum effect. In other words, the wavelength of the laser light 3a is controlled to be 664nm and 650nm when NPe6 and PH-1126 are used as the photosensitizer, respectively. The controlling operation has been described earlier in the first embodiment.

When the laser light 3a is irradiated to the target portion 5, the focus A is selectively treated by the action of the photosensitizer accumulated there beforehand. At the same time, the photosensitizer in the focus A is excited by the laser light 3a and consequently emits fluorescence of the specified wavelength as described before. The target portion 5 is thus diagnosed by analyzing the image of fluorescence. The fluorescence shows the wavelength considerably approximate to that of the irradiating laser light 3a and has the weak intensity, and therefore is strongly influenced by the scattering light of the laser light 3a. It is hence conventionally generally difficult to pick up and analyze the image of fluorescence.

As such, the fluorescence is guided through the band-pass filter 27 via the image transmission line 23 according to the instant embodiment. More specifically, the fluorescence is passed through the band-pass filter 27 which has such characteristic as exemplified in Fig. 3 and allows only the fluorescence emitted from the using photosensitizer to pass while shutting the irradiating laser light 3a, whereby the influences of the scattering light of the laser light 3a are eliminated. As a result, the image of fluorescence alone is inputted to the image-picking-up/analyzing unit 25. The image-picking-up/analyzing unit 25 picks up and analyzes the image data of fluorescence, the result of which is displayed at the image displaying unit 26 such as a television and recorded in a recording unit such as a VTR. By observing the display, the focus A can be diagnosed in real time even in the middle of the treatment.

It is also possible to control and shift the wavelength of the irradiating laser light 3a from that of the fluorescence with an aim to improve the separation of the fluorescence (S) from the scattering light (N) of the irradiating laser light 3a (S/N ratio). That is, the irradiating laser light 3a may be controlled to be shifted from the center wavelength in the absorption band of the using photosensitizer (e.g., 664nm or 650nm when NPe6 or PH-1126 is used) to be away from the wavelength of the fluorescence within the absorption band (e.g., 660nm or 644nm when NPe6 or PH-1126 is used). If the wavelength of the irradiating laser light 3a is controlled as above, the S/N ratio is improved. At the same time, since the energy of the irradiating laser light 3a is kept within the absorption band of the photosensitizer as described earlier in the first embodiment, the curing effect is hardly deteriorated.

The oscillating wavelength of the laser light 3 can be made variable also in a diagnosing/curing apparatus which uses only a specific photosensitizer (for instance, NPe6 or PH-1126) within the range of the effective absorption band of the photosensitizer (e.g., 664±5nm or 650±10nm in the case of NPe6 or PH-1126).

Since the wavelength of the irradiating laser light 3a can be controlled easily in the medical laser apparatus of the embodiments of the present invention, even when the concurrent diagnosis with the treatment becomes unnecessary, it is possible to return the wavelength of the irradiating laser light 3a to the center wavelength of the absorption band of the photosensitizer, that is, the optimum wavelength for the treatment. Moreover, it is an advantage of the medical laser apparatus of the embodiments to display whether the wavelength of the laser light 3 conforms to the controlling condition. If the wavelength of the laser light 3 is not in compliance with the controlling condition, the laser light is shut as mentioned before.

Accordingly, owing to the band-pass filter provided in the diagnosing/curing apparatus of the embodiments, it becomes possible to execute diagnosis and treatment concurrently by a single laser light source. The wavelength of the laser light is controlled by the wavelength controlling unit to be away from the wavelength of the fluorescence emitted by the photosensitizer within the absorption band of the photosensitizer, so that the S/N ratio ensuring stable images during the concurrent diagnosis with the treatment is satisfied.

Although the laser in the first embodiment is a semiconductor laser, the other kinds of lasers can be employed so long as the full width at half maximum is narrow and the oscillating wavelength of the laser light is variable. Needless to say, the semiconductor laser 1 is not limited to the one having the characteristic described in the first embodiment. For example, the laser can be made up of an external resonating type semiconductor laser which wavelength is controlled by change of resonance from its external.

Further, although the temperature is controlled in the arrangement of feedback control by means of the wavelength detecting unit 9 in the first embodiment, it is possible to control the wavelength correctly if a memory means storing the relationship between the temperature and the oscillating wavelength of the using semiconductor laser is provided to control the temperature of the semiconductor laser based on the relationship.

As is fully described hereinabove, the medical laser apparatus is provided with the laser as a light source and the wavelength controlling unit for the laser. This laser emits the laser light of the narrow full width at half maximum and the oscillating wavelength of the laser light is variable. Therefore, the medical laser apparatus is able to achieve the oscillating wavelength suitable for the kind of the using photosensitizer as well as the exciting condition of the photosensitizer, and thus efficiently excite the photosensitizer. Moreover, the medical laser apparatus is advantageously almost maintenance-free, compact and inexpensive.

According to the diagnosing/curing apparatus of the present invention, since the image of fluorescence is displayed by the image displaying unit also during the treatment of the focus, it becomes possible to diagnose and cure the focus by a single light source, and to make diagnosis of the focus during the treatment in the simple and compact structure. The diagnosing/curing apparatus is easy to handle.

Although the present invention has been fully described in connection with the preferred embodiments thereof with reference to the accompanying drawings, it is to be noted that various changes and modifications are apparent to those skilled in the art. Such changes and modifications are to be understood as included within the scope of the present invention as defined by the appended claims.

## Claims

1. "A medical laser apparatus for a method of diagnosing and/or curing a focus which has preliminarily been treated with a photosensitizer (6) having an affinity to the focus, by irradiating the focus with light from a laser (1) and detecting the fluorescent light collected from the excited focus,
**characterized in that**
- the laser (1) is a semiconductor laser,
- the medical laser apparatus comprises controlling means (8), wherein the wavelength of the laser (1) is controllable by the controlling means (8) controlling the temperature of the laser (1), and the laser is such that
- the wavelength of the laser (1) is variable within 650±10 nm; or
- within 664±5 nm; and
the laser (1) has a full width at half maximum which is narrower than the width of the band, in which the energy absorption of the photosensitizer is equal to or more than 90% of the maximal value of energy absorption in the vicinity of 650 or 664 nm, respectively."

2. The medical laser apparatus as claimed in claim 1, which is provided with a memory means for storing the relationship between the temperature and the wavelength of the semiconductor laser (1), whereby the temperature of the semiconductor laser is controlled based on the relationship.

3. The medical laser apparatus as claimed in claim 1, wherein the laser (1) is an external resonating type semiconductor laser.

4. The medical laser apparatus as claimed in any one of claims 1 through 3, further comprising a control means (7) for deciding whether or not the wavelength of the laser light projected from the semiconductor laser (1) conforms to a wavelength controlling condition, and a wavelength displaying means (10) for displaying the decision outputted from the control means.

5. The medical laser apparatus as claimed in any one of claims 1 through 3, further comprising a control means (7) for deciding whether or not the wavelength of the laser light projected from the semiconductor laser (1) conforms to a wavelength controlling condition, and an automatic irradiation stopping means (7, 11) for controlling not to irradiate the laser light when the wavelength of the laser light projected from the semiconductor laser is not in conformity with a wavelength controlling condition.

6. A medical laser apparatus as claimed in claim 1, further comprising
a light transmission line (22) for guiding the laser light projected from the medical laser apparatus to the vicinity of a focus to be diagnosed and/or cured;
an image transmission line (23) for guiding fluorescence light emitted from a photosensitizer excited by the laser light to observe the focus and a periphery thereof;
a fluorescence separating means (27) for separating only the fluorescence; and
an image-picking-up/analyzing means (25) for picking up and analyzing an image of the fluorescence light obtained by the fluorescence separating means.

7. The medical laser apparatus as claimed in claim 6, further comprising an image displaying means (26) for displaying an analyzed result of the image of fluorescence obtained by the image-picking-up/analyzing means, so that the image of fluorescence is displayed by the image displaying means during treatment of the focus.

8. The medical laser apparatus as claimed in claim 6 or 7, wherein the fluorescence separating means (27) is a band-pass filter allowing the fluorescence to pass therethrough and blocking the irradiating laser light.

9. The medical laser apparatus as claimed in claim 1, wherein the wavelength controlling means (8) shifts the wavelength of the laser light away from the wavelength of fluorescence emitted from the photosensitizer within the absorption band of the photosensitizer at the treatment of the focus concurrently performed with diagnosis of the focus with the use of the displayed image of fluorescence.

10. The medical laser apparatus as claimed in claim 9 for the use with a chlorin photosensitizer, wherein the wavelength of the laser light is controlled to a wavelength of 660 nm, when variable within 664±5 nm and wherein a band-pass filter (27) for passing the fluorescence passes the wavelength 670 nm and blocks the wavelength 660 nm.

11. The medical laser apparatus as claimed in claim 9 for the use with a pheophorbide photosensitizer, wherein the wavelength of the laser light is controlled to a wavelength of 644 nm when variable within 650±10 nm, and wherein a band-pass filter (27) for passing the fluorescence passes the wavelength 654 nm and blocks the wavelength 644 nm.

## Patentansprüche

1. Medizinischer Laser für ein Verfahren der Diagnostik und / oder Therapie eines Herdes, welcher vorbereitend mit einem Photosensibilisator (6) behandelt wurde, der eine Affinität zu dem Herd aufweist, durch das Bestrahlen des Herdes mit Licht mit einem Laser (1) und das Erfassen des fluoreszierenden Lichts, welches von dem angeregten Herd gesammeit wird,
**dadurch gekennzeichnet, dass**
- der Laser (1) ein Halbleiterlaser ist,
- der medizinische Laser Steuerungsmittel (8) umfasst, wobei die Wellenlänge des Lasers (1) durch das Steuerungsmittel (8) steuerbar ist, welches die Temperatur des Lasers (1) steuert, und
der Laser so ausgebildet ist, dass
- die Wellenlänge des Lasers (1) innerhalb von 650 ± 10 nm oder
- innerhalb von 664 ± 5 nm variabel ist; und
der Laser (1) eine Halbwertsbreite aufweist, die schmaler ist als die Breite des Bandes, in dem die Energieabsorption des Photosensibilisators gleich oder höher ist als 90% des Höchstwertes der Energieabsorption nahe der Werte von jeweils 650 beziehungsweise 664 nm.

2. Medizinischer Laser nach Anspruch 1, welcher mit einem Speicher zum Speichern des Verhältnisses zwischen der Temperatur und der Wellenlänge des Halbleiterlasers (1) ausgestattet ist, wobei die Temperatur des Halbleiterlasers auf Basis dieses Verhältnisses gesteuert wird.

3. Medizinischer Laser nach Anspruch 1, wobei der Laser (1) ein Halbleiterlaser des Typs mit einem externen Resonator ist.

4. Medizinischer Laser nach einem der Ansprüche 1 bis 3, welcher ferner ein Steuerungsmittel (7) umfasst, zum Entscheiden, ob die Wellenlänge des von dem Halbleiterlaser (1) ausgestrahlten Laserlichts mit einem Wellenlängen-Steuerzustand übereinstimmt oder nicht, und eine Wellenlängen-Anzeige (10) zum Anzeigen der Entscheidung, die von dem Steuerungsmittel ausgegeben wird.

5. Medizinischer Laser nach einem der Ansprüche 1 bis 3, welcher ferner ein Steuerungsmittel (7) umfasst, zum Entscheiden, ob die Wellenlänge des von dem Halbleiterlaser
(1) ausgestrahlten Laserlichts mit einem Wellenlängen-Steuerzustand übereinstimmt oder nicht, und eine automatische Einrichtung zur Unterbrechung der Bestrahlung (7, 11) zum Steuern des Nichtausstrahlens des Laserlichts, wenn die Wellenlänge des von dem Halbleiterlaser (1) ausgestrahlten Laserlichts nicht mit einem Wellenlängen-Steuerzustand übereinstimmt.

6. Medizinischer Laser nach Anspruch 1, weicher weiterhin folgendes umfasst:
- eine Lichtübertragungsfeitung (22) zum Leiten des von dem medizinischen Lasers ausgestrahlten Laserlichts in die Nähe eines zu diagnostizierenden und / oder therapierenden Herdes;
- eine Bildübertragungsleitung (23) zum Leiten eines von einem durch das Laserlicht angeregten Photosensibilisator emittierten Fluoreszenzlichts, um den Herd und eine Umgebung von diesem zu beobachten;
- ein Fluoreszenz-Filter (27) zum Herausfiltern nur der Fluoreszenz; und
- ein Bild-Aufnahme-/Analysemittel (25) zum Aufnehmen und Analysieren eines Bildes des Fluoreszenzlichts, weiches durch den Fluoreszenz-Filter erzielt wurde.

7. Medizinischer Laser nach Anspruch 6, welcher ferner eine Bildanzeige (26) umfasst, zum Anzeigen eines analysierten Ergebnisses des Bildes des Fluoreszenzlichts, welches durch das Bild-Aufnahme-/Analysemittel erzielt wurde, so dass das Bild des Fluoreszenzlichts während der Behandlung des Herdes durch die Bildanzeige angezeigt wird.

8. Medizinischer Laser nach Anspruch 6 oder 7, wobei der Fluoreszenz-Filter (27) ein Bandpassfilter ist, welcher der Fluoreszenz gestattet hindurchzutreten und das ausgestrahlte Laserlicht abfängt.

9. Medizinischer Laser nach Anspruch 1, wobei das Wellenlängen-Steuerungsmittel (8) die Wellenlänge des Laserlichts bei der Behandlung des Herdes, die gleichzeitig mit der Diagnose des Herdes ausgeführt wird, unter Verwendung des angezeigten Bildes des Fluoreszenzlichts innerhalb des Absorptionsbereichs des Photosensibilisators von der Wellenlänge der von dem Photosensibilisator ausgestrahlten Fluoreszenz weg verschiebt.

10. Medizinischer Laser nach Anspruch 9 zur Verwendung mit einem Chlor-Photosensibilisator, wobei die Wellenlänge des Laserlichts auf eine Wellenlänge von 660 nm eingestellt wird, variable innerhalb eines Bereichs von 664 ± 5 nm, und wobei ein Bandpassfilter (27) zum Hindurchtreten der Fluoreszenz die Wellenlänge von 670 nm hindurchtreten lässt und die Wellenlänge von 660 nm blockiert.

11. Medizinischer Laser nach Anspruch 9 zur Verwendung mit einem Pheophorbid-Photosensibilisator, wobei die Wellenlänge des Laserlichts auf eine Wellenlänge von 644 nm eingestellt wird, variabel innerhalb eines Bereichs von 650 ± 10 nm, und wobei ein Bandpassfilter (27) zum Hindurchtreten der Fluoreszenz die Wellenlänge von 654 nm hindurchtreten lässt und die Wellenlänge von 644 nm blockiert.

## Revendications

1. Laser médical destiné à un procédé de diagnostic et/ou de traitement d'un foyer qui a été traité préalablement au moyen d'un photosensibilisateur (6) ayant une affinité avec le foyer, par irradiation du foyer par de la lumière délivrée par un laser (1) et détection de la lumière fluorescente captée à partir du foyer excité,
**caractérisé en ce que**
- le laser (1) est un laser à semi-conducteur,
- le laser médical comprend un moyen de commande (8), dans lequel on peut commander la longueur d'onde du laser (1) à l'aide du moyen de commande (8) en commandant la température du laser (1), et le laser est tel que
- la longueur d'onde du laser (1) est variable dans les limites de 650 ± 10 nm ; ou
- dans les limites de 664 ± 5 nm ; et
le laser (1) a une pleine largeur à la moitié du maximum qui est plus étroite que la largeur de la bande, dans laquelle l'absorption d'énergie du photosensibilisateur est égale ou supérieure à 90 % de la valeur maximale de l'absorption d'énergie dans le voisinage respectivement de 650 ou de 654 nm.

2. Laser médical selon la revendication 1, qui est muni d'un moyen de mémoire destiné à mémoriser la relation entre la température et la longueur d'onde du laser (1) à semi-conducteur, grâce à quoi on commande la température du laser (1) à semi-conducteur en se basant sur la relation.

3. Laser médical selon la revendication 1, dans lequel le laser (1) est un laser à semi-conducteur du type à résonance extérieure.

4. Laser médical selon l'une quelconque des revendications 1 à 3, comprenant en outre un moyen de commande (7) destiné à décider si la longueur d'onde de la lumière laser projetée par le laser (1) à semi-conducteur est ou non conforme à une condition de commande de la longueur d'onde, et un moyen (10) d'affichage de longueur d'onde destiné à afficher la décision délivrée par le moyen de commande.

5. Laser médical selon l'une quelconque des revendications 1 à 3, comprenant en outre un moyen de commande (7) destiné à décider si la longueur d'onde de la lumière laser projetée par le laser (1) à semi-conducteur est ou non conforme à une condition de commande de la longueur d'onde, et un moyen automatique (7, 11) d'arrêt de l'irradiation destiné à commander l'absence d'irradiation de la lumière laser si la longueur d'onde de la lumière laser projetée par le laser à semi-conducteur n'est pas en conformité avec une condition de commande de la longueur d'onde.

6. Laser médical selon la revendication 1, comprenant en outre
une ligne (22) de transmission de lumière destinée à guider la lumière laser projetée par le laser médical vers le voisinage d'un foyer à diagnostiquer et/ou à traiter ;
une ligne (23) de transmission d'image destinée à guider la lumière fluorescente émise par un photosensibilisateur excité par la lumière laser afin d'observer le foyer et une périphérie de celui-ci ;
un moyen (27) de séparation de la fluorescence destiné à séparer seulement la fluorescence ; et
un moyen (25) de saisie et d'analyse d'image destiné à saisir et à analyser une image de la lumière fluorescente obtenue par le moyen de séparation de la fluorescence.

7. Laser médical selon la revendication 6, comprenant en outre un moyen (26) d'affichage d'image destiné à afficher un résultat d'analyse de l'image de la fluorescence obtenue par le moyen de saisie et d'analyse d'image, de manière à ce que l'image de la fluorescence soit affichée par le moyen d'affichage d'image pendant le traitement du foyer.

8. Laser médical selon la revendication 6 ou 7, dans lequel le moyen (27) de séparation de la fluorescence est un filtre passe-bande permettant à la fluorescence d'y passer et bloquant la lumière laser d'irradiation.

9. Laser médical selon la revendication 1, dans lequel le moyen (8) de commande de la longueur d'onde décale la longueur d'onde de la lumière laser à distance de la longueur d'onde de la fluorescence émise par le photosensibilisateur dans la bande d'absorption du photosensibilisateur lors du traitement du foyer exécuté simultanément avec le diagnostic du foyer au moyen de l'image de la fluorescence affichée.

10. Laser médical selon la revendication 9, destiné à l'utilisation avec un photosensibilisateur au chlore, dans lequel la longueur d'onde de la lumière laser est commandée selon une longueur d'onde de 660 nm variable dans les limites de 664 ± 5 nm, et dans laquelle un filtre passe-bande (27) destiné au passage de la fluorescence laisse passer la longueur d'onde de 670 nm et bloque la longueur d'onde de 660 nm.

11. Laser médical selon la revendication 9, destiné à l'utilisation avec un photosensibilisateur au phéophorbide, dans lequel la longueur d'onde de la lumière laser est commandée selon une longueur d'onde de 644 nm variable dans les limites de 650 ± 10 nm, et dans laquelle un filtre passe-bande (27) destiné au passage de la fluorescence laisse passer la longueur d'onde de 654 nm et bloque la longueur d'onde de 644 nm.
